(19) Europäisches Patentamt
European Patent Office
Office européen des brevets

(11) Publication number: **0 201 342 B1**

# (12) EUROPEAN PATENT SPECIFICATION

(45) Date of publication of the patent specification: **01.08.90**

(51) Int. Cl.[5]: **A61K 7/06**

(21) Application number: **86303527.5**

(22) Date of filing: **09.05.86**

## (54) Hair-conditioning resin compositions.

(30) Priority: **10.05.85 JP 99350/85**

(43) Date of publication of application: **17.12.86 Bulletin 86/46**

(45) Publication of the grant of the patent: **01.08.90 Bulletin 90/31**

(84) Designated Contracting States: **BE CH DE FR GB IT LI**

(56) References cited:
**AT-B-286 504**
**GB-A-1 031 540**
**US-A-3 026 250**
**US-A-3 934 595**
**US-A-4 015 612**
**US-A-4 030 512**
**US-A-4 358 567**

(73) Proprietor: **MITSUBISHI PETROCHEMICAL CO., LTD., 5-2, Marunouchi 2-chome, Chiyoda-ku Tokyo(JP)**

(72) Inventor: **Kubota, Ikuo, 5-1-602, Akasaka 7-chome Minato-ku, Tokyo(JP)**
Inventor: **Hayama, Kazuhide, C/o Mitsubishi Yuka Fine Chemicals Co., Ltd., No 1, Toho-cho Yokkaichi-shi Mie(JP)**
Inventor: **Narazaki, Kanji, C/o Mitsubishi Yuka Fine Chemicals Co., Ltd., No 1, Toho-cho Yokkaichi-shi Mie(JP)**

(74) Representative: **Diamond, Bryan Clive et al, Gee & Co., Chancery House, Chancery Lane, London WC2A 1QU(GB)**

EP 0 201 342 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel hair-conditioning resin compositions having hair cosmetic properties such as softening, moderate moistening and wavesetting, and the ability to impart voluminousness and to facilitate combing out.

It has already been proposed to use quaternary ammonium salt compounds or quaternary ammonium salt-series polymers in hair-conditioning cosmetics, such as shampoos, rinses, hair treatments and wavesetting lotions, as disclosed in U.S. Patents 3,934,595, 4,030,512, 3,836,537 and 4,075,131; and in Japanese Patent Publication Nos. 11128/80 and 17009/80.

Among the known quaternary ammonium salt compounds are stearyl-trimethylammonium chloride, lauryl-trimethylammonium chloride and distearyl-dimethylammonium chloride. And among the known quaternary ammonium salt-series polymers are dimethylaminoethyl methacrylate-series quaternary ammonium salt polymers, diallyl-dimethylammonium chloride-series polymers and cationic celluloses.

The quaternary ammonium salt compounds have the double advantage of making the hair easier to comb and of softening it: however, they have the disadvantages of reducing the voluminousness of the hair and settlement of the hair, and of making it more difficult to set. The quaternary ammonium salt-series polymers have the advantage of making the hair easier to comb: however, they have the disadvantage of imparting a specific malaise to the hair, that is, the accumulation of the polymers on the hair by the repetitious use thereof, known as the build-up problem, impairs the good feeling of the hair. Furthermore, these quaternary ammonium salt compounds and polymers have other disadvantages in their incompatibility with anion-charged compounds caused by their cationic charge and the hyperhygroscopicity of the quaternary ammonium salt group; in the feel of the hair at high temperature and humidity; in the worsening of the hair's condition; and in the rapid losing of the hair's wavesetting property.

On the other hand, although being useful for setting the hair, the zwitterionic polymers according to the prior art are not useful for conditioning hair.

It is therefore an object to eliminate or reduce the disadvantages of the prior art preparations.

We have found that certain zwitterionic copolymers exhibit an excellent hair-conditioning effect.

US-A 4 358 567 discloses a resin composition with wave-setting property which is prepared by zwitterionizing with a halo acetate a co-polymer resin which is prepared from a mixture containing 25 to 45 wt.% of tertiary amine-containing meth(acrylate), 5 to 65 wt.% of C 4–24 meth(acrylate), and optionally 0 to 50 wt.% of C 4–24 meth(acrylate), and optionally 0 to 50 wt.% of C 1–3 meth(acrylate) and of other optional monomers.

We have devised an improved composition of this type which has a higher proportion of the tertiary amine-containing meth(-acrylate).

According to the present invention there is provided a hair-conditioning resin composition prepared by modifying a copolymer resin with a zwitterionizing agent, said copolymer resin being prepared by copolymerizing in a hydrophilic solvent in the presence of an initiator a polymerizable mixture containing

(A) 65–90% by weight of a polymerizable vinyl monomer having the following general formula (I):

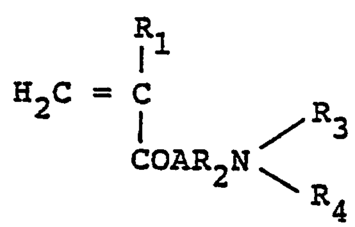

(I)

wherein $R_1$ represents a hydrogen atom or a methyl group, $R_2$ represents an alkylene group having from 1 to 4 carbon atoms, $R_3$ and $R_4$ each represents an alkyl group having from 1 to 4 carbon atoms, and A represents an oxygen atom or an NH group,

(B) 10 - 35% by weight of polymerizable vinyl monomer having the following general formula (II):

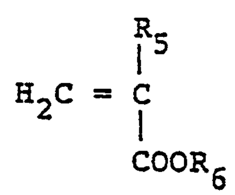

(II)

wherein $R_5$ represents a hydrogen atom or a methyl group, and $R_6$ represents a saturated or unsaturated alkyl group having from 12 to 24 carbon atoms, and

(C) 0 - 25% by weight of other polymerizable vinyl monomer, said zwitterionizing agent having the following general formula (III)

$$XR_7COOB \quad ... \quad (III)$$

wherein $R_7$ represents an alkylene group having from 1 to 4 carbon atoms, X represents a bromine, chlorine or iodine atom, and B represents an alkali metal ion, an ammonium salt or an amine salt.

On occasion, the preparation of the resin composition of the invention preferably includes the final steps of filtering any precipitate, and purifying with ion-exchange resin.

Monomer (A) is a derivative of acrylic acid or methacrylic acid represented by the above-mentioned general formula (I). Hereinafter, both the acrylic acid and methacrylic acid are generally referred to as "(meth)acrylic acid". In the above formulae (I) to (III), $R_1$ - $R_7$, A and B represent substituents as defined above, and particularly, it is preferred that $R_1$ is a methyl group, $R_2$ is an alkyl group having 2 to 3 carbon atoms, $R_3$ and $R_4$ are each a methyl group or an ethyl group, and A is an oxygen atom. Examples of preferred Monomer (A) include dimethylamino-ethyl (meth)acrylate, dimethylamino-propyl (meth)acrylate, diethylamino-ethyl (meth)acrylate, dimethylamino-ethyl (meth)acrylamide and diethylaminopropyl (meth)acrylamide.

The proportion of Monomer (A) to the total monomer content is 65 - 90% by weight, and preferably, 70 - 85% by weight. If the proportion is less than 65% by weight, the resulting zwitterionic copolymer films are hard, thereby making the hair stiff and more difficult to comb, and reducing its moisture content. If the proportion exceeds 90% by weight, the resulting copolymer films are tacky, thereby making the hair heavy, reducing its voluminousness and impairing its ability to settle.

Monomer (B) is a (meth)acrylic ester represented by the above general formula (II), in which it is generally preferred that $R_5$ is a methyl group and $R_6$ is an alkyl group having from 16 to 20 carbon atoms.

If $R_6$ is an alkyl group having less than 12 carbon atoms, the resulting zwitterionic copolymer films are hard, thereby making the hair stiff and more difficult to comb, and reducing its moisture content. Examples of Monomer (B) include palmityl (meth)acrylate, stearyl (meth)acrylate, oleyl (meth)acrylate, myristyl (meth)acrylate and behenyl (meth)acrylate.

The proportion of Monomer (B) to the total monomer content is 10 - 35% by weight, and preferably, 15 - 30% by weight. If the proportion is less than 10% by weight, the resulting copolymer films are tacky, thereby making the hair heavy and losing its voluminousness and settlement ability. If the proportion exceeds 35% by weight, the resulting copolymer films are hard, thereby making the hair stiff and more difficult to comb, and imparting thereto the above-mentioned malaise.

Other vinyl monomer may be added as component (C) to Monomers (A) and (B) in order to give moderate hardness and flexibility to the resulting films as necessary. Examples of the other vinyl monomer include methyl (meth)acrylate, isobutyl (meth)acrylate, cyclohexyl (meth)acrylate, 2-ethylhexyl (meth)acrylate, acrylonitrile, acrylamide, styrene, vinyl acetate, hydroxyethyl (meth)acrylate, (meth)acrylic ester of polyethylene glycol or polypropylene glycol and N-vinyl-pyrrolidone. The proportion of the other vinyl monomer to the total monomer content is 0 - 25% by weight.

Specific examples of the zwitterionizing agent represented by the above-mentioned general formula (III) include sodium monobromoacetate, potasium monochloroacetate, lithium monochloropropionate, neutral matter of monochloroacetic acid and ammonia, 2-amino-2-methyl-1-propanol, triethanolamine and 2-mino-2-ethyl-1,3-propanediol.

The proportion of the zwitterionizing agent to Monomer (A) (or to the total of Monomer (A) and other Monomer (C) in the case that the other monomer component (C) contains nitrogen atoms to be zwitterionized) is 0.7 -1.3, preferably 0.8 - 1.2, mol per mol of Monomer (A) or Monomer (A) and (C).

The above-mentioned Monomers (A), (B) and (C) are copolymerized in a hydrophilic solvent. The term "hydrophilic solvent" used herein means an organic solvent having a solubility of 10 [g/100 g water/25°C] or more in water. Preferred examples of the hydrophilic organic solvent used in the present invention include methanol, ethanol, isopropanol, ethylene glycol, ethylcellosolve, dioxane, methyl acetate and dimethylformamide. The hydrophilic organic solvent may be in the hydrous state as long as no trouble is thereby incurred during polymerization, filtration and the like. An example of hydrous organic solvent is 95% ethanol.

Copolymerization is carried out by a conventional solution polymerization method, for example one comprising the steps of dissolving the monomers in the solvent, adding an initiator thereto, and heating the mixture with stirring under nitrogen stream. Preferred examples of the initiator include peroxides, such as benzoyl peroxide, a lauroyl peroxide and azo compounds, such as azo-bis-isobutyronitrile.

The copolymerization may be carried out simultaneously or partially and progressively with respect to all the kinds and mounts of monomers.

It is preferred that the solvent is used in an amount such that the concentration of the resulting copolymer solution lies within the range from 30 to 70% by weight.

The polymerization conditions, such as the method of introducing the monomers and initiator, and the amount of the solvent used, are suitably selected. It is, however, preferred that the conditions are determined so that the resulting copolymer has a numerical average molecular weight of from 30,000 to 500,000.

The zwitterionizing reaction can be carried out by adding a hydrophilic solvent solution or suspension of the zwitterionizing agent completely or partially to the copolymer solution after the polymerization, and heating at a temperature of from about 70 to about 90°C for a time of from about 4 to about 12 hours with suitable stirring in an inactive atmosphere, for example, in a flow of nitrogen gas.

Where B in the general formula (III) is an alkali metal such as sodium, potassium or lithium, an inorganic salt (BX) is precipitated with the progression of the zwitterionizing reaction; the precipitated inorganic salt is removed. Removal of the precipitated inorganic salt is performed by any solid-liquid separation method such as centrifugal separation or filtration.

The zwitterionized copolymer solution obtained by such methods of removing the inorganic salt may still contain a little (0.1 - 1% by weight) of inorganic salt. To remove such a small remaining amount of inorganic salt, the zwitterionized copolymer solution can be treated by batch-type or flow-type ion exchange. Thereby, the inorganic salt content can be reduced to a value of 0.1% by weight or less.

Where B in the general formula (III) is an amine or an ammonium salt, an organic salt (BX) is not precipitated with the progression of the zwitterionizing reaction. Accordingly, the zwitterionized polymer solution can be directly used as a homogeneous solution without the steps of separating and removing BX.

The thus obtained resin can be used, in the form of a hydrophilic solvent solution, of a solution from which the solvent has been removed or of a solution in which the hydrophilic solvent has been replaced by pure water, in cosmetics for hair.

The resin according to the present invention is compatible with and may be added to a hair cosmetic such as a shampoo, rinse, hair treatment or wavesetting lotion, in order to give hair conditioning effects. The proportion of the resin to the cosmetic ranges from 0.01 to 5% by weight as zwitterionic polymer.

The present invention will now be illustrated in more detail in the following Examples thereof.

EXAMPLE 1

70 parts by weight of dimethylamino-ethyl methacrylate, 10 parts by weight of myristyl methacrylate, 20 parts by weight of stearyl methacrylate, and 50 parts by weight of ethanol were introduced into a five neck polymerization flask, equipped with a reflux cooler, a dropping funnel, a thermometer, a glass inlet for nitrogen substitution, and a stirrer. 0.6 part by weight of α,α'-azo-bis-isobutyronitrile was added thereto and the mixture was refluxed with heating at 80°C in a flow of nitrogen gas for 4 hours to thus carry out copolymerization.

At the end of the copolymerization, an ethanol suspension containing 40% by weight of potassium monochloroacetate equimolar with the dimethylamino-ethyl methacrylate was added to the flask, drop by drop, by means of the dropping funnel. The mixture was heated at 80°C in a flow of nitrogen gas for 10 hours to carry out the zwitterionizing reaction.

A precipitate was separated from the obtained viscous suspension by the use of a pressure filter (made by Nippon Dying Machine Mfg. Co., Ltd.).

The filtrate was passed through a column filled with recovered cation-exchange resin (tradename "DIAION PK-220"; in which, after recovery the system was subject to substitution of ethanol) and then passed through a column filled with recovered anion-exchange resin (tradename "DIAION PA-416"; in which, after recovery the system was subject to substitution of ethanol) to thus carry out purification.

The thus obtained light-yellow transparent solution was diluted with ethanol so as to adjust the copolymer resin content thereof to 30% by weight.

The thus obtained copolymer resin solution was subject to measurement of numerical average molecular weight by an osmotic pressure method (at 25°C) by using an osmometer (made by Parkin-Elmer Inc.), and the resultant numerical average molecular weight was 300,000.

The resin ethanol solution was diluted 60 times with pure water. The diluted solution was applied to a 23 cm long and 2 g weight bundle of hair. The hair was then dried with exposure to air. The hair-conditioning effect of the solution was excellent. (See Table 1.)

Next, 3 g of the resin ethanol solution were introduced to 100 g of an aqueous solution containing 20% by weight of alcohol sulfate sodium salt to thus obtain a shampoo liquid. The shampoo liquid was applied to a 23 cm long and 2 g weight bundle of hair. The hair was then washed with water and dried with exposure to air. The hair-conditioning effect of the liquid was excellent. (See Table 1.)

EXAMPLES 2 - 4

Resin solutions were prepared in the same manner as described in Example 1 except that the monomers used in Example 1 were replaced by monomers shown in Table 1. The resulting resin solutions were then evaluated in the same manner described in Example 1. The hair-conditioning effect of the respective solution was excellent. (See Table 1.)

EXAMPLE 5

Copolymerization was carried out in the same manner as described in Example 1 except that the monomers used in Example 1 were replaced by monomers shown in Table 1. At the end of the copolymerization, an ethanol suspension containing 40% by weight of neutral matter of monochloroacetic acid and 2-amino-2-methyl-1-propanol equimolar with the dimethylamino-ethyl methacrylate was added to the polymerization flask, drop by drop, by means of the dropping funnel. The mixture was heated at 80°C for 8 hours to thus carry out the zwitterionizing reaction.

The thus obtained solution was diluted with ethanol so as to adjust the resin content thereof to 30% by weight. The resulting resin solution was then evaluated in the same manner as described in Example 1. The hair-conditioning effect of the solution was excellent. (See Table 1.)

EXAMPLE 6

A resin solution was prepared in the same manner as described in Example 5 except that the monomers used in Example 5 were replaced by monomers shown in Table 1 and the zwitterionizing agent was replaced by neutral matter of monochloroacetic acid and triethanolamine. The resulting resin solution was then evaluated in the same manner as described in Example 1. (See Table 1.)

COMPARATIVE EXAMPLES 1 - 4

Resin solutions were prepared in the same manner described in Example 1 except that the monomers used in Example 1 were replaced by monomers shown in Table 2. The resulting resin solutions were then evaluated in the same manner as described in Example 1. The resin solutions were inferior in stiffness, combing-out, and so on, that is, they were unsatisfactory as hair-conditioning resins. (See Table 2.)

COMPARATIVE EXAMPLE 5

A resin solution was prepared in the same manner as described in Example 1 except that the ethanol used for the polymerization was used in the amount of 150 parts by weight. The resulting resin solution was then evaluated in the same manner as described in Example 1. The resin solution was inferior in tackiness, combing-out, and so on, that is, it was unsatisfactory as hair-conditioning resin. (See Table 2.)

Measurement of molecular weight was made on the copolymer resin in the same manner as described in Example 1. The resin had a numerical average molecular weight of 20,000.

COMPARATIVE EXAMPLE 6

70 parts by weight of dimethylamino-ethyl methacrylate, 10 parts by weight of myristyl methacrylate, 20 parts by weight of stearyl methacrylate, and 45 parts by weight of ethanol were introduced into the same flask as described in Example 1 and heated to 70°C in a flow of nitrogen gas. A solution obtained by dissolving 0.3 part by weight of α,α'-azo-bis-isobutyronitrile into 5 parts by weight of ethanol was added to the flask having been heated to 70°C, drop by drop, for 4 hours. After the dropwise addition, the temperature was maintained at 70°C for 3 hours to thus obtain a copolymer. After the end of the zwitterionization, the process performed in Example 1 was followed.

The resulting resin solution was then evaluated in the same manner as described in Example 1. The resin solution was inferior in combing-out and stiffness, that is, it was unsatisfactory as hair-conditioning resin. (See Table 2.)

Measurement of molecular weight was made on the copolymer resin in the same manner as described in Example 1. The resin had a numerical average molecular weight of 600,000.

The performance of the respective resin shown in Tables 1 and 2 was evaluated with organolepic test and the types thereof were classified into the following three.

o : Good
Δ : Unsatisfactory
x : Very bad

TABLE 1

| Example | Composition (% by weight) Monomers (A) | | Monomers (B) | | Other Monomers (C) | | Performance Pure water Dilution Volumi-nousness | Stiffness | Combing-out | Tackiness | Shampoo – Model Volumi-nousness | Stiffness | Combing-out | Tacki-ness |
|---|---|---|---|---|---|---|---|---|---|---|---|---|---|---|
| 1 | Dimethyl-amino-ethyl methacrylate | 70 | Myristyl methacrylate | 10 | | | o | o | o | o | o | o | o | o |
| | | | Stearyl methacrylate | 20 | | | | | | | | | | |
| 2 | Diethyl-amino-ethyl methacrylate | 85 | Behenyl methacrylate | 15 | | | o | o | o | Δ | Δ | o | o | o |
| 3 | Dimethyl-amino-pro-pyl acryl-amide | 70 | Stearyl methacrylate | 30 | | | o | o | o | o | o | o | o | o |
| 4 | Dimethyl-amino-ethyl methacrylate | 65 | Stearyl methacrylate | 20 | Polypropyl-ene glycol (n=9) meth-acrylic acid ester | 15 | o | Δ | o | o | o | o | o | o |
| 5 | Dimethyl-amino-ethyl methacrylate | 70 | Stearyl methacrylate | 20 | | | o | o | o | o | o | o | o | o |
| | | | Stearyl acrylate | 10 | | | | | | | | | | |
| 6 | Dimethyl-amino-pro-pyl meth-acrylate | 75 | Behenyl methacrylate | 15 | Butyl methacrylate | 10 | o | o | o | o | o | o | o | o |

TABLE 2

| Comp. Example | Composition (% by weight) Monomers (A) | | Monomers (B) | | Other Monomers (C) | | Performance Pure water Dilution Volumi-nousness | Stiffness | Combing-out | Tackiness | Shampoo – Model Volumi-nousness | Stiffness | Combing-out | Tacki-ness |
|---|---|---|---|---|---|---|---|---|---|---|---|---|---|---|
| 1 | Diethyl-amino-ethyl methacrylate | 60 | Myristyl methacrylate | 20 | | | o | Δ | Δ | Δ | o | o | Δ | Δ |
| | | | Lauryl methacrylate | 20 | | | | | | | | | | |
| 2 | Dimethyl-amino-ethyl methacrylate | 50 | | | Methyl methacrylate | 25 | o | x | x | o | o | x | x | o |
| | | | | | Isobutyl methacrylate | 25 | | | | | | | | |
| 3 | Dimethyl-amino-propyl meth-acrylate | 50 | Tridecyl methacrylate | 30 | Ethyl methacrylate | 20 | o | x | x | o | o | x | x | o |
| 4 | Dimethyl-amino-ethyl methacrylate | 40 | Stearyl methacrylate | 20 | Polypropyl-ene glycol (n=9) meth-acrylic acid ester | 20 | o | x | x | o | o | x | x | o |
| | | | | | Methyl methacrylate | 20 | | | | | | | | |
| 5 | Dimethyl-amino-ethyl methacrylate | 70 | Myristyl methacrylate | 10 | | | x | o | Δ | Δ | x | o | Δ | Δ |
| | | | Stearyl methacrylate | 20 | | | | | | | | | | |
| 6 | Dimethyl-amino-ethyl methacrylate | 70 | Myristyl methacrylate | 10 | | | o | x | x | o | Δ | Δ | x | o |
| | | | Stearyl methacrylate | 20 | | | | | | | | | | |

## Claims

1. A hair-conditioning resin composition prepared by modifying a copolymer resin with a zwitterionizing agent, characterised in that said copolymer resin is prepared by copolymerizing in a hydrophilic solvent in the presence of an initiator a polymerizable mixture containing

(A) 65 - 90% by weight of a polymerizable vinyl monomer having the following general formula (I):

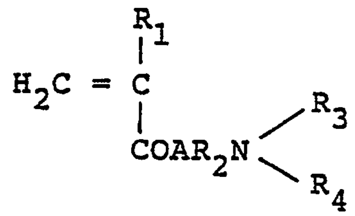

(I)

wherein $R_1$ represents a hydrogen atom or a methyl group, $R_2$ represents an alkylene group having from 1 to 4 carbon atoms, $R_3$ and $R_4$ each represent an alkyl group having from 1 to 4 carbon atoms, and A represents an oxygen atom or an NH group,

(B) 10 - 35% by weight of polymerizable vinyl monomer having the following general formula (II):

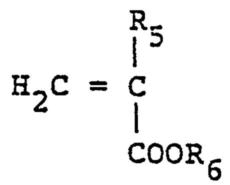

(II)

wherein $R_5$ represents a hydrogen atom or a methyl group, and $R_6$ represents a saturated or unsaturated alkyl group having from 12 to 24 carbon atoms, and

(C) 0–25% by weight of other polymerizable vinyl monomer, selected from methyl (meth)acrylate, isobutyl (meth)acrylate, cyclohexyl (meth)acrylate, 2-ethylhexyl (meth)acrylate, acrylnitrile, acrylamide, styrene, vinyl acetate, hydroxyethyl (meth)acrylate, (meth)acrylic esters of polyethylene glycol or polypropylene glycol and N-vinyl-pyrrolidone,

and in that said zwitterionizing agent has the following general formula (III):

$$XR_7COOB \quad (III)$$

wherein $R_7$ represents an alkylene group having from 1 to 4 carbon atoms, X represents a bromine, chlorine or iodine atom, and B represents an alkali metal ion, an ammonium salt or an amine salt.

2. A composition as claimed in claim 1, in which said resin has a numerical average molecular weight of from 30,000 to 500,000.

3. A composition as claimed in claim 1 or 2, in which in the general formula (I) $R_1$ represents a methyl group, $R_2$ an alkyl group having 2 or 3 carbon atoms, $R_3$ and $R_4$ a methyl or ethyl group, and A an oxygen atom.

4. A composition as claimed in claim 1 or 2, in which said monomer (A) is selected from dimethylamino-ethyl methacrylate, diethylamino-ethyl methacrylate, dimethylamino-propyl acrylamide and dimethylamino-propyl methacrylate.

5. A composition as claimed in any preceding claim, in which in the general formula (II) $R_5$ is a methyl group and $R_6$ an alkyl group having from 16 to 20 carbon atoms.

6. A composition as claimed in any one of claims 1 to 4, in which said monomer (B) is selected from myristyl methacrylate, stearyl methacrylate, behenyl methacrylate, and stearyl acrylate.

7. A composition as claimed in any preceding claim, in which said zwitterionizing agent is selected from potassium monochloroacetate, neutral matter of monochloroacetic acid and 2-amino-2-methyl-1-propanol, and neutral matter of monochloroacetic acid and triethanolamine.

## Patentansprüche

1. Harzzusammensetzung für die Haarkonditionierung, hergestellt durch Modifizierung eines Copolymerharzes mit einem Zwitterionisierungsmittel, dadurch gekennzeichnet, daß das genannte Copolymerharz hergestellt wird, indem in einem hydrophilen Lösungsmittel eine polymerisierbare Mischung in Gegenwart eines Initiators copolymerisiert wird, wobei die Mischung

(A) 65–90 Gew.-% eines polymerisierbaren Vinylmonomers mit der folgenden allgemeinen Formel (I)

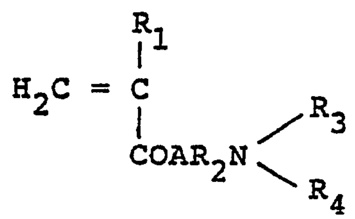

(I)

worin $R_1$ ein Wasserstoffatom oder eine Methylgruppe darstellt, $R_2$ eine Alkylengruppe mit 1 bis 4 Kohlenstoffatomen darstellt, $R_3$ und $R_4$ je eine Alkylgruppe mit 1 bis 4 Kohlenstoffatomen darstellen und A ein Sauerstoffatom oder eine NH-Gruppe bedeuten,

(B) 10–35 Gew.-% eines polymerisierbaren Vinylmonomers mit der folgenden allgemeinen Formel (II)

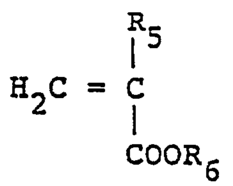

(II)

worin $R_5$ ein Wasserstoffatom oder eine Methylgruppe darstellt und $R_6$ eine gesättigte oder ungesättigte Alkylgruppe mit 12 bis 24 Kohlenstoffatomen bedeutet und

(C) 0–25 Gew.-% eines anderen polymerisierbaren Vinylmonomers, ausgewählt aus Methylmethacrylat, Isobutylmethacrylat, Cyclohexylmethacrylat, 2-Ethylhexylmethacrylat, Acrylonitril, Acrylamid, Styrol, Vinylacetat, Hydroxyethylmethacrylat, Methacrylsäureester von Polyethylenglykol oder Polypropylenglykol und N-Vinyl-pyrrolidon enthält und

daß das genannte Zwitterionisierungsmittel folgende allgemeine Formel (III) aufweist

$XR_7COOB$ (III)

worin $R_7$ eine Alkylengruppe mit 1 bis 4 Kohlenstoffatomen bedeutet, X ein Brom-, Chlor- oder Iodatom ist und B ein Alkalimetallion, ein Ammoniumsalz oder Aminsalz darstellt.

2. Zusammensetzung gemäß Anspruch 1, worin das genannte Harz ein mittleres, numerisches Molekulargewicht von 30 000 bis 500 000 aufweist.

3. Zusammensetzung gemäß Anspruch 1 oder 2, worin in der allgemeinen Formel (I) $R_1$ eine Methylgruppe, $R_2$ eine Alkylgruppe mit 2 bis 3 Kohlenstoffatomen, $R_3$ und $R_4$ eine Methyl- oder Ethylgruppe und A ein Sauerstoffatom bedeuten.

4. Zusammensetzung gemäß Anspruch 1 oder 2, worin das genannte Monomer (A) ausgewählt ist aus Dimethylamino-ethylmethacrylat, Diethylamino-ethylmethacrylat, Dimethylamino-propylacrylat und Dimethylamino-propylmethacrylat.

5. Zusammensetzung gemäß einem der vorhergehenden Ansprüche, worin in der allgemeinen Formel (II) $R_5$ eine Methylgruppe und $R_6$ eine Alkylgruppe mit 16 bis 20 Kohlenstoffatomen ist.

6. Zusammensetzung gemäß einem der Ansprüche 1 bis 4, worin das genannte Monomer (B) ausgewählt ist aus Myristylmethacrylat, Stearylmethacrylat, Behenylmethacrylat und Stearylacrylat.

7. Zusammensetzung gemäß einem der vorhergehenden Ansprüche, worin das genannte Zwitterionisierungsmittel ausgewählt ist aus Kaliummonochloracetat, neutralem Material aus Monochlor-essigsäure und 2-Amino-2-methyl-1-propanol und neutrales Material aus Monochlor-essigsäure und Triethanolamin.

**Revendications**

1. Composition de résine pour le conditionnement des cheveux préparée par modification d'une résine copolymère avec un agent de zwitterionisation, caractérisée en ce qu'on prépare la résine copolymère en copolymérisant dans un solvant hydrophile en présence d'un initiateur un mélange susceptible de se polymériser contenant

(A) un monomère du vinyle polymérisable à raison de 65 - 90% en poids ayant la formule générale (I) suivante:

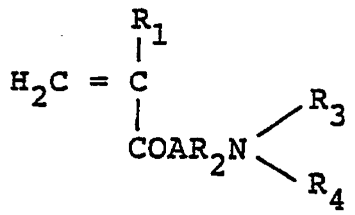

(I)

dans laquelle $R_1$ représente un atome d'hydrogène ou un groupe méthyle, $R_2$ représente un groupe alkylène ayant de 1 à 4 atomes de carbone, $R_3$ et $R_4$ représentent chacun un groupe alkyle ayant de 1 à 4 atomes de carbone et A représente un atome d'oxygène ou un groupe NH,
(B) un monomère du vinyle polymérisable à raison de 10 à 35% en poids ayant la formule générale (II) suivante:

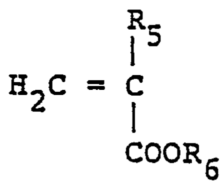

(II)

dans laquelle $R_5$ représente un atome d'hydrogène ou un groupe méthyle, et $R_6$ représente un groupe alkyle saturé ou non-saturé ayant de 12 à 24 atomes de carbone, et
(C) un autre monomère du vinyle polymérisable à raison de 0 - 25 % en poids, choisi entre le (méth)acrylate de méthyle, le (méth)acrylate d'isobutyle, le (méth)acrylate de cyclohexyle, le (méth)acrylate de 2-éthylhexyle, l'acrylonitrile, l'acrylamide, le styrène, l'acétate de vinyle, le (méth)acrylate d'hydroxyéthyle, les esters (méth)acryliques de polyéthylène-glycol ou de polypropylène-glycol et la N-vinyl-pyrrolidone, et en ce que ledit agent de zwitterionisation a la formule générale (III) suivante:

$$XR_7COOB \quad (III)$$

dans laquelle $R_7$ représente un groupe alkylène ayant de 1 à 4 atomes de carbone, X représente un atome de brome, de chlore ou de iode et B représente un ion de métal alcalin, un sel d'ammonium ou un sel aminé.

2. Composition selon la revendication 1, dans laquelle ladite résine a un poids moléculaire moyen numérique allant de 30'000 à 500'000.

3. Composition selon la revendication 1 ou la revendication 2, dans laquelle $R_1$ dans la formule générale (I) représente un groupe méthyle, $R_2$ un groupe alkyle ayant 2 ou 3 atomes de carbone, $R_3$ et $R_4$ un groupe méthyle ou éthyle, et A un atome d'oxygène.

4. Composition selon la revendication 1 ou la revendication 2, dans laquelle ledit monomère (A) est choisi entre le méthacrylate de diméthylamino-éthyle, le méthacrylate de diéthylamino-éthyle, l'acrylamide de diméthylamino-propyle et le méthacrylate de diméthylamino-propyle.

5. Composition selon l'une quelconque des revendications précédentes, dans laquelle $R_5$ dans la formule générale (II) est un groupe méthyle et $R_6$ un groupe alkyle ayant de 16 à 20 atomes de carbone.

6. Composition selon l'une quelconque des revendications 1 à 4, dans laquelle ledit monomère (B) est choisi entre le méthacrylate de myristyle, le méthacrylate de stéaryle, le méthacrylate de behenyle et l'acrylate de stearyle.

7. Composition selon l'une quelconque des revendications précédentes, dans laquelle ledit agent de zwitterionisation est choisi entre le monochloroacétate de potassium, une substance neutre de l'acide monochloroacétique et du 2-amino-2-méthyl-1-propanol et une substance neutre de l'acide monochloroacétique et de la triéthanolamine.